# EUROPEAN PATENT APPLICATION

(11) **EP 4 230 737 A1**
(43) Date of publication of application: **23.08.2023**
(21) Application number: 21869551.8
(22) Date of filing: 13.08.2021
(51) Int. Cl.: C12N 15/10, C12N 9/22, C07K 14/47, A61K 48/00, A01K 67/027

(54) **NOVEL ENHANCED BASE EDITING OR REVISING FUSION PROTEIN AND USE THEREOF**

(30) Priority: 21.09.2020 KR 20200121730
(71) Applicant: Korea University Research and Business Foundation, Seoul 02841 (KR)
(72) Inventor: KIM, Kyoungmi, Seoul 03024 (KR); YOON, Da Eun, Uijeongbu-si, Gyeonggi-do 11741 (KR)
(74) Representative: Henderson, Helen Lee
(86) International application number: PCT/KR2021/010785
(87) International publication number: WO 2022/059928

(57) **Abstract**

The present invention relates to a fusion protein developed by adding configurational elements, such as chromatin-modulating peptides, etc., and modifying the arrangement thereof, on the basis of conventional developed base editors. The fusion protein of the present invention can be provided as a novel base editor which exhibits base editing efficiency due to the inclusion of CMP and is free of the occurrence of undesired random base insertion and deletion due to the employment of deadCas9 and as such, can be expected to find advantageous applications in the field of genetic engineering for various purposes, such as exquisite gene therapy, construction and research of transgenic animal models, etc.

## Description

### Technical Field

The present disclosure relates to a fusion protein developed by adding configurational elements, such as chromatin-modulating peptides (CMP) and varying the arrangement thereof on the basis of conventional developed base editors, in which the fusion protein of the present disclosure can be provided as a novel base editor which improves base editing efficiency due to the inclusion of CMP and is free of the occurrence of undesired random base insertion and deletion using deadCas9.

### Background Art

More than 75,000 pathogenic mutations cause human genetic diseases, and about 50% of the genetic diseases caused by the pathogenic mutations are induced by point mutations. With the development of a CRISPR system, a homology-directed repair (HDR) method using donor DNA has been proposed as a therapeutic approach, but its efficiency is very low, and its application is limited. In order to overcome the limitations of the HDR method, a base editor (BE) that may be controlled at the base level was developed, and recently, a prime editor (PE) composed of nCas9 and reverse transcriptase, which is a new precise genome editing tool, was developed as a gene reviser. The prime editor has an advantage of overcoming the low HDR efficiency of the Cas9 system and capable of substitution of C→A, C→G, G→C, G→T, A→C, A→T, T→A, and T→G, but its operability has not yet been verified in various organisms. Therefore, it is inevitable to use a base editor together with a prime editor for gene editing.

The base editors developed based on the CRISPR system include a cytosine base editor (CBE) and an adenine base editor (ABE), and the CBE and the ABE are capable of efficient substitution of C→T, T→C, A→G, and G→A in a variety of organisms. In addition, through a recent study, CGBE1 has been reported as C-to-G base editors capable of base revising from C to G in human cells. However, generation of precise target mutations such as insertion, substitution, or deletion of one or more bases has a problem of low efficiency by intracellular HDR. To improve the problem, various base editor variants have been developed, among them, AncBE4max and ABEmax show higher nucleotide substitution capacities for most targets than BE3 and ABE, but the editing efficiency of AncBE4max is only 69 to 77% and the editing efficiency of ABEmax is only 27 to 52%, so that the task of improving efficiency still remains.

On the other hand, xBE3 and xABE were developed to improve compatibility with various PAMs, but show low editing efficiency in most targets despite the NGG PAM sequence, and thus, up to now, AncBE4max and ABEmax are proposed as optimal base editors for clinical and biological research. However, as described above, there remain a problem of enhancing the editing efficiency of AncBE4max and ABEmax, undesired insertion and deletion of additional base sequences, and an off-target problem, and the present inventors have intensively researched to develop a base editor which enhanced editing efficiency and was free of an off-target problem and undesired base insertion and deletion problems and then completed the present disclosure.

### [Related Art Document] [Non-Patent Document] Int J Mol Sci. 2020 Aug 28;21(17):6240

### Disclosure of the Invention

### Technical Goals

An object of the present disclosure is to provide a novel base editor with enhanced base editing efficiency by further including chromatin modulating peptides (CMPs) to conventionally developed base editors.

In addition, another object of the present disclosure is to provide a novel base editor in which a frequency of occurrence of random base insertion and deletion is significantly reduced by using deadCas9 instead of nickaseCas9 together with the addition of the CMPs.

In addition, yet another object of the present disclosure is to provide a gene editing composition based on the novel base editor, a gene editing viral vector, a method for gene editing using the same, and a method for constructing a transfected cell line and a gene-modified mammal using the same.

However, technical objects of the present disclosure are not limited to the aforementioned purpose and other objects which are not mentioned may be clearly understood to those skilled in the art from the following description.

### Technical Solutions

According to an embodiment of the present disclosure, there is provided a fusion protein as a base editor which is provided to a CRISPR/Cas9 system to enhance base editing efficiency.

The fusion protein of the present disclosure may include one or more chromatin-modulating peptides (CMPs) together with a Cas9 protein, the CMP may enhance base editing efficiency by improving the accessibility of the base editor to the chromatin, and the CMP may be at least one selected from the group consisting of a high-mobility group nucleosome binding domain 1 (HN1), a histone H1 central globular domain (H1G), and combinations thereof.

In one embodiment of the present disclosure, the fusion protein may be provided as a cytosine base editor (CBE) including cytosine deaminase, or provided as an adenine base editor (ABE) including tRNA adenosine deaminase (TadA).

In another embodiment of the present disclosure, when the fusion protein is provided as the CBE including cytosine deaminase, the cytosine deaminase may be apolipoprotein B mRNA editing enzyme, catalytic polypeptide-like (APOBEC), and the Cas9 protein is preferably dead Cas9 (dCas9) in which an RuvC domain and an HNH domain are deactivated. The dCas9 may function as an accurate base editor by significantly decreasing the occurrence frequency of undesired base insertion and/or deletion, that is, random indel induced by a conventional CBE. Meanwhile, the decrease in base editing efficiency due to the use of dCas9 may be recovered by the addition of CMPs proposed in the present disclosure.

Meanwhile, the fusion protein provided as the CBE may significantly reduce the occurrence of random indel by using dCas9, but the random indel that still occur may be completely eliminated through dCas9 bound with deaminase.

Accordingly, the fusion protein provided as the CBE may further include an uracil DNA-glycosylase inhibitor (UGI) peptide, and the UGI peptide may be directly linked to a C-terminus of dCas9, one or more UGI peptides linked to dCas9 may be included, and the fusion protein designed and experimentally confirmed by the present inventors may include two UGI peptides.

As yet another embodiment of the present disclosure, the fusion protein may further include a nuclear localization signals (NLS) peptide, and the NLS peptide may be located at an N-terminus and a C-terminus of the fusion protein, but in the NLS peptide at the C-terminus, the location of the CMP to be bound may be variable.

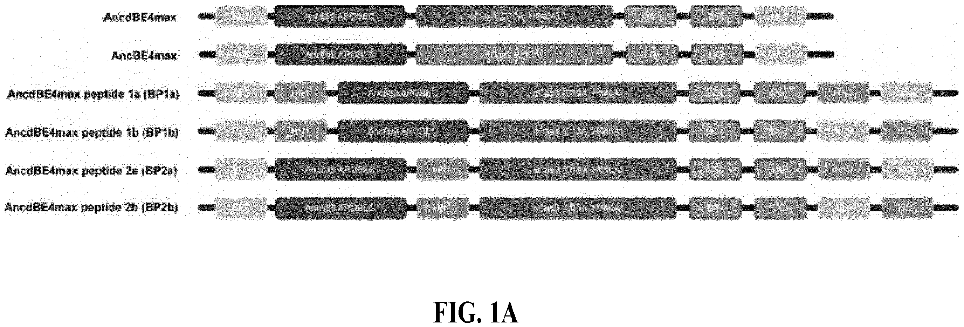

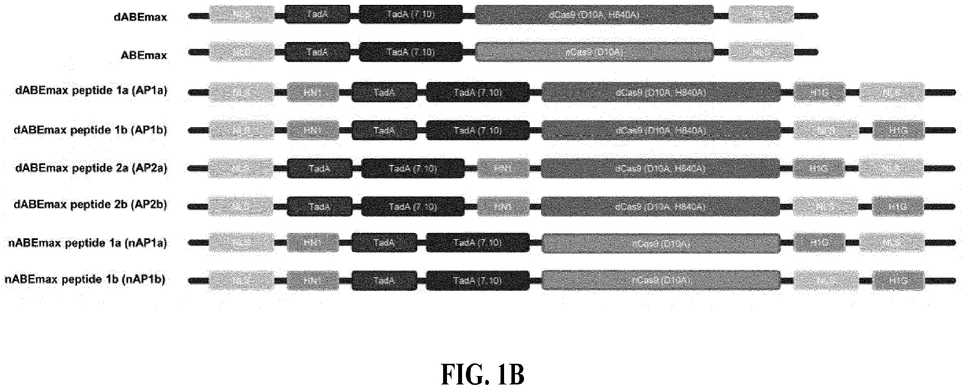

More specifically, [NLS peptide]-[APOBEC]-[dCas9 protein]-[NLS peptide] may be located in the order from the N-terminus to the C-terminus of the fusion protein, and the UGI peptide may be directly linked to a dCas9 C-terminus, NH1 may be located at an N-terminus or C-terminus of APOBEC, and HIG may be located at the C-terminus of the UGI peptide or the C-terminus of the fusion protein (see AncdBE4max variant, and peptides 1a-b and 2a-b in FIG. 1).

As yet another embodiment of the present disclosure, when the fusion protein is provided as the ABE including TadA, the Cas9 protein may be a Cas9 protein, that is, dCas9 or nickase Cas9 (nCas9) in which the RuvC domain and/or the HNH domain are deactivated.

Since ABEmax, the most optimized ABE developed to date, does not have a high occurrence frequency of random indel, the ABE variant of the present disclosure may be provided as a base editor with enhanced target base editing efficiency by including nCas9 as it is and additionally including CMPs.

The structure of the fusion protein of the present disclosure provided as the ABE may have [NLS peptide]-[TadA]-[nCas9 or dCas9 protein]-[NLS peptide] in the order from the N-terminus to the C-terminus, and HN1 may be located at the N-terminus or the C-terminus of TadA and H1G may be located at the C-terminus of the fusion protein or the C-terminus of the Cas9 protein.

In addition, according to another embodiment of the present disclosure, there is provided a gene editing composition and kit including the fusion protein, plasmid DNA or mRNA encoding the fusion protein, or a vector including the plasmid DNA or mRNA; and single guide RNA (sgRNA) hybridizing with an off-target DNA strand to induce cleavage of a target DNA strand, a plasmid capable of expressing the sgRNA, or a vector including the plasmid, so as to use the fusion protein in a CRISPR/Cas9 system.

As an embodiment of the present disclosure, the vector may be one or more selected from the group consisting of an adenovirus vector, adeno-associated virus (AAV), lentivirus, and combinations thereof.

In addition, according to yet another embodiment of the present disclosure, there is provided a method for gene editing including bringing the gene editing composition into contact with a target region including a target nucleic acid sequence in vitro or ex vivo.

In addition, according to still another embodiment of the present disclosure, there is provided a lentiviral vector including mRNA encoding the fusion protein and single guide RNA (sgRNA).

In addition, according to still yet another embodiment of the present disclosure, there is provided a method for constructing a transfected cell line including introducing the gene editing composition or the lentiviral vector into a mammalian cell and a transfected cell line constructed by the method.

In addition, according to still yet another embodiment of the present disclosure, there is provided a method for constructing a gene-modified mammalian animal including introducing the gene editing composition or the lentiviral vector into a mammalian cell to obtain a gene-modified mammalian cell; and transplanting the obtained gene-modified mammalian cell into the fallopian tube of a mammalian foster mother.

As one embodiment of the present disclosure, the mammalian cell may be a mammalian embryonic cell.

### Effects

According to the embodiments of the present disclosure, it is confirmed that it is possible to enhance base editing efficiency using chromatin-modulating peptides (CMPs) and significantly reduce random indel in the case of using dead Cas9 instead of nickase Cas9, and it is possible to provide a base editor with significantly enhanced genome editing efficiency and target specificity. In addition, according to the present disclosure, a mutant animal model of a target gene is constructed to confirm transmission of the mutation to the next generation, a phenotypic change, and the like. Therefore, the gene editing composition including the improved prime editor according to the present disclosure is expected to be usefully used for various applications, such as the production and research of humanized animal models, a field of genetic engineering technology, and the treatment means of genetic diseases.

### Brief Description of Drawings

FIG. 1A illustrates structures of CBE variants, and FIG. 1B illustrates structures of ABE variants.

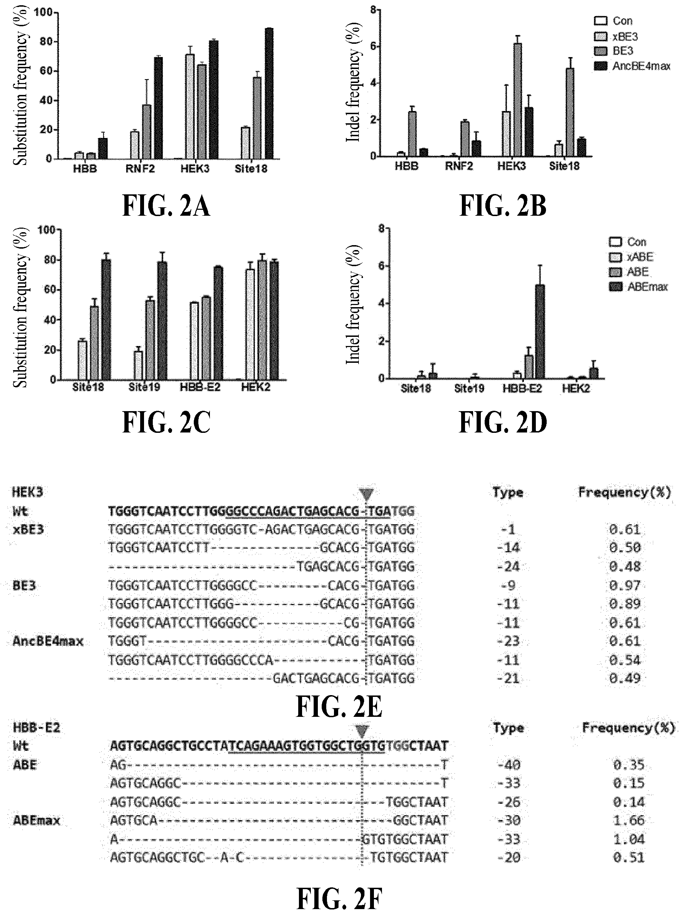

FIG. 2 illustrates confirming the activity of conventional base editors, and comparing and confirming the base editing efficiency and random indel occurrence frequencies of CBE variants (A and B) and ABE variants (C and D) targeting human genes. Each data showed an average value through three repeated experiments. In addition, FIG. 2E illustrates indel patterns by the CBE variants and FIG. 2F illustrates indel patterns generated by the ABE variants. Red arrows and dotted lines indicate cleavage sites of spCas9.

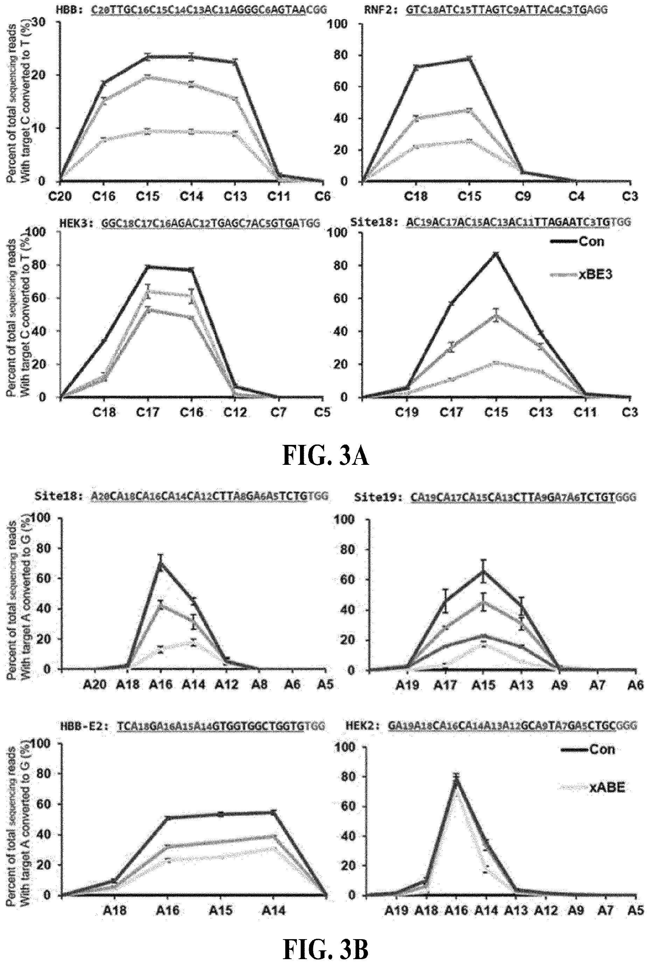

FIG. 3 illustrates confirming the base editing efficiency of C or A targets in active windows, in which FIG. 3A shows the editing efficiency for each editing window of human targets *HBB, RNF2, HEK3* and *Site18* for CBE variants in HEK293 cells, and FIG. 3B shows the editing efficiency for each editing window of human targets *Site18, Site19, HBB-E2* and *HEK2* for ABE variants in HEK293 cells. Each data is presented as the mean ± S.D of three repeated experiments, a Pam region is marked in blue, a target sequence is marked with an underline, and a substituted target region is marked in red.

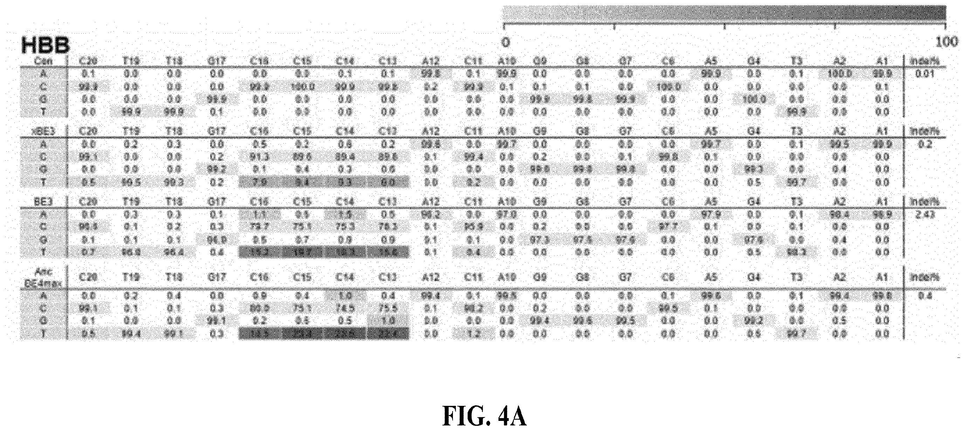

FIGS. 4A to 4D confirm base substitution efficiency at each location of a CBE target sequence. A CBE variant induces a C→T substitution in 13 to 18 nt upstream of a PAM sequence, and C to T conversion is highlighted in blue. The yellow box represents an originally intended target sequence, and the pink box represents conversion (C→A or C→G) of an off-target base. Each data was expressed as an average value of three repeated experiments.

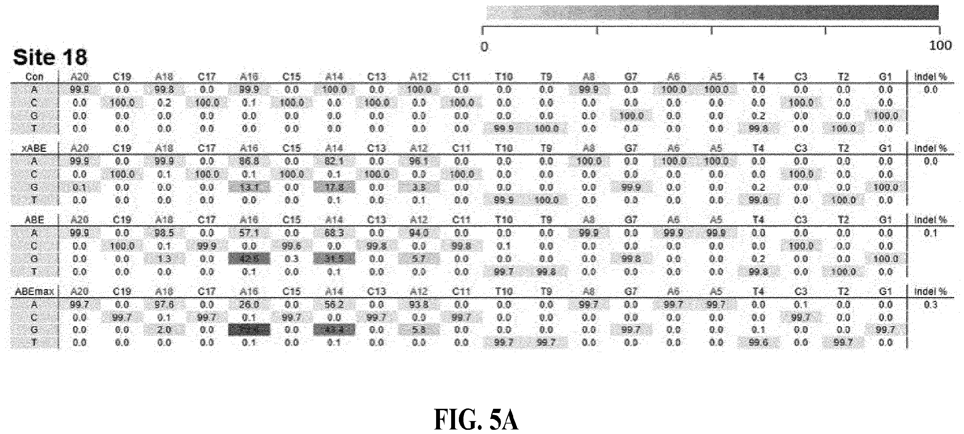

FIGS. 5A to 5D confirm base substitution efficiency at each location of an ABE target sequence. An ABE variant induces an A→G substitution 13 to 18 nt upstream of the PAM sequence, and A to G conversion is highlighted in red. The yellow box represents an originally intended target sequence, and each data was expressed as an average value of three repeated experiments.

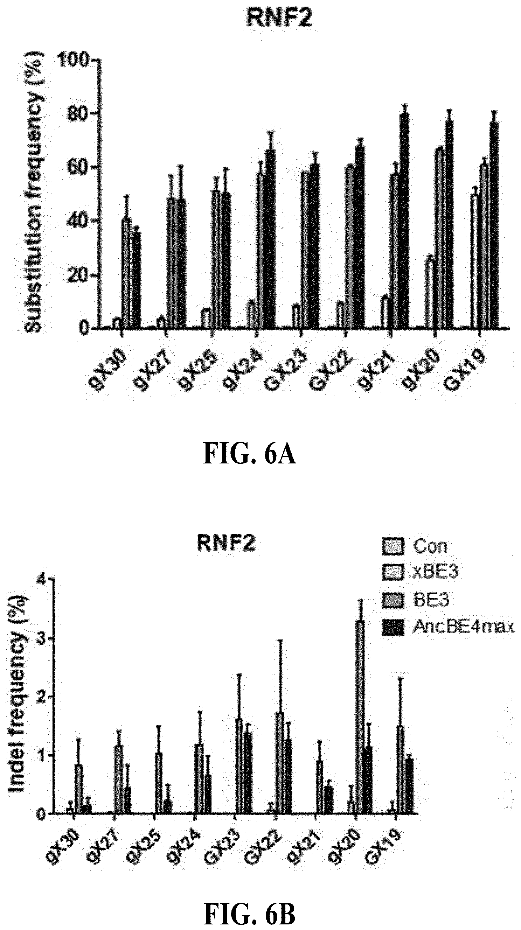

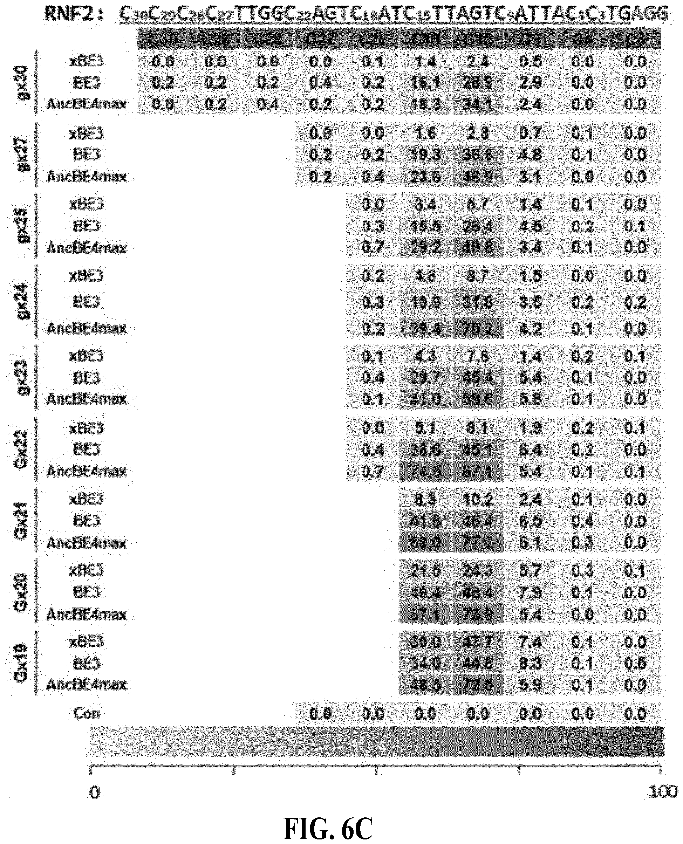

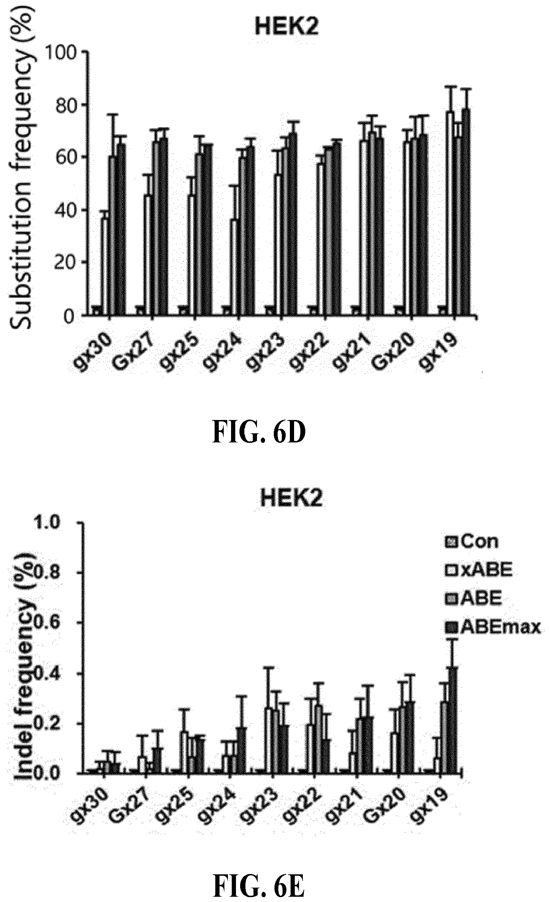

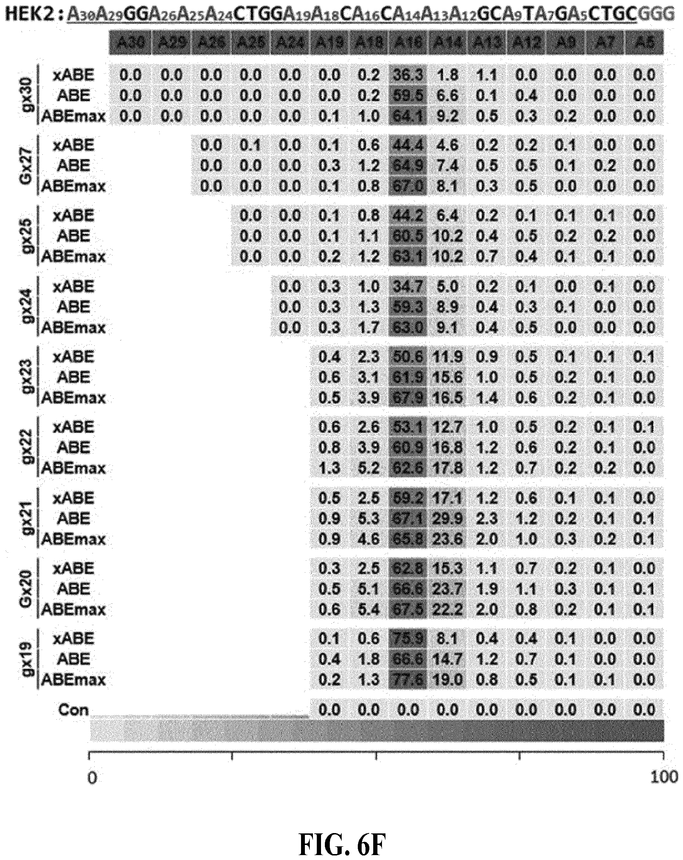

FIG. 6 illustrates confirming changes in specificity and editing efficiency of the editing window at a target location according to an sgRNA length. FIGS. 6A and 6B illustrate confirming the conversion efficiency of target bases of CBE variants according to a length of sgRNA, FIG. 6C shows target base conversion efficiency of CBE variants according to an sgRNA length in *RNF2,* FIGS. 6D and 6E illustrate confirming the conversion efficiency of target bases of ABE variants according to a length of sgRNA, and FIG. 6F shows target base conversion efficiency of ABE variants according to an sgRNA length in *HEK2.* Each base substitution frequency was analyzed by targeted deep sequencing, and all data were expressed as mean ± SD of three repeated experiments.

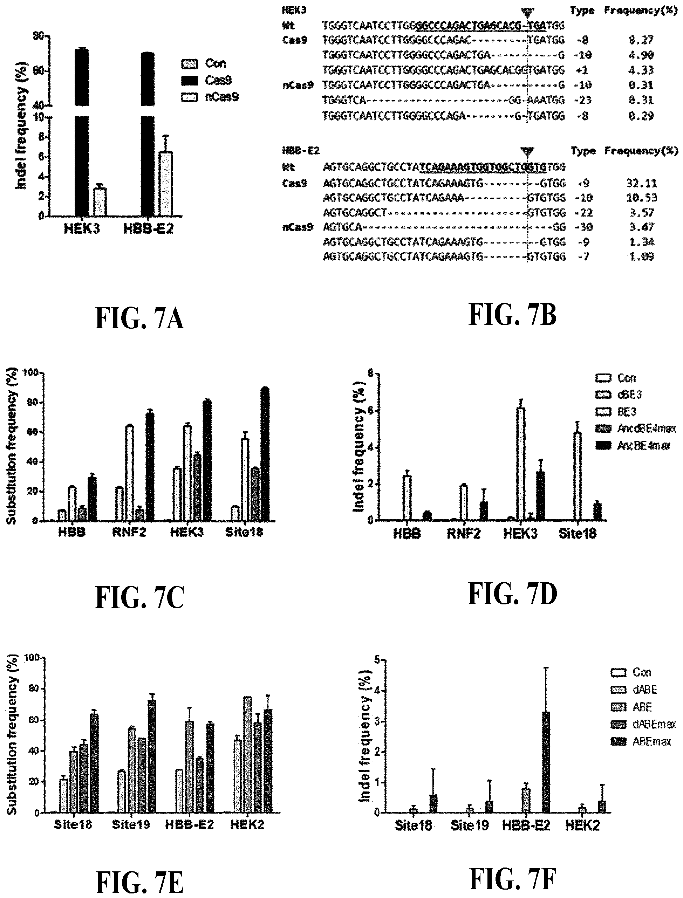

FIG. 7 illustrates confirming that a base editor using nCas9 reduces the occurrence frequency of random indel compared to a base editor using Cas9, and a base editor using dCas9 more remarkably reduces the occurrence frequency of indel than a base editor using nCas9. Each data was expressed as mean ± SD of three repeated experiments.

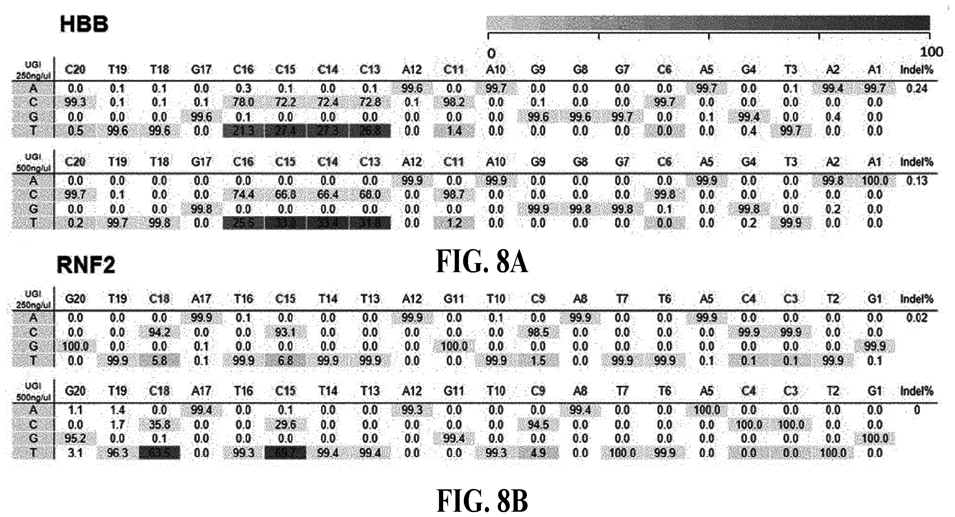

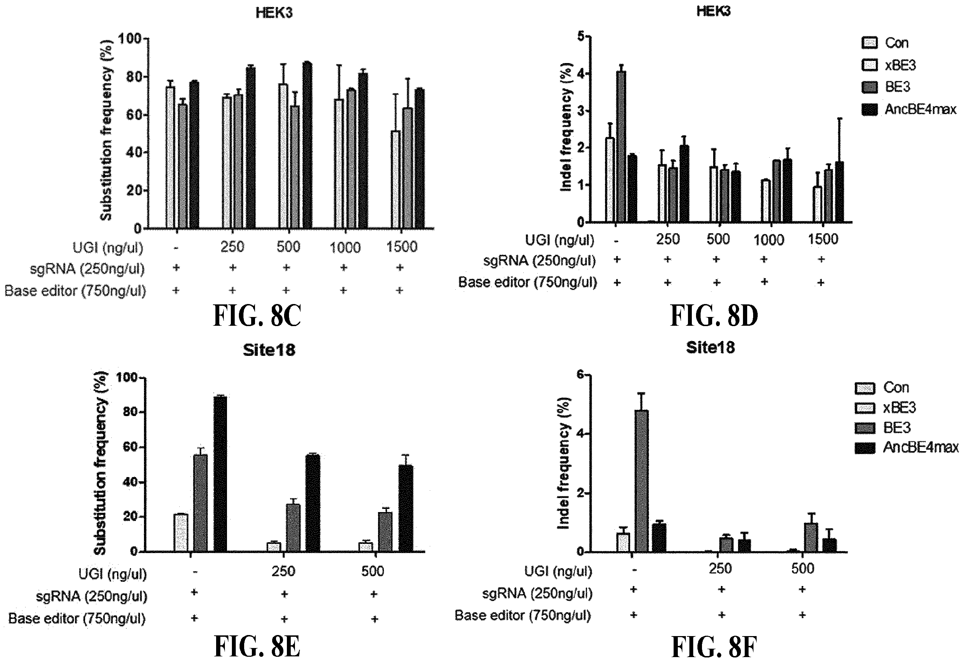

FIG. 8 illustrates confirming that the addition of UGI eliminates random indel occurring in CBE variants, in which FIG. 8A illustrates base substitution efficiency of CBE variants at a target site of a target gene, and FIG. 8B illustrates substitution efficiency of the target base and the occurrence frequency of random indel in target genes *HEK3* and *Site18* of the CBE variant according to a concentration of additional UGI treatment. The random indel and the base conversion frequency were confirmed by performing targeted deep sequencing, and each data was expressed as mean ± SD of three repeated experiments.

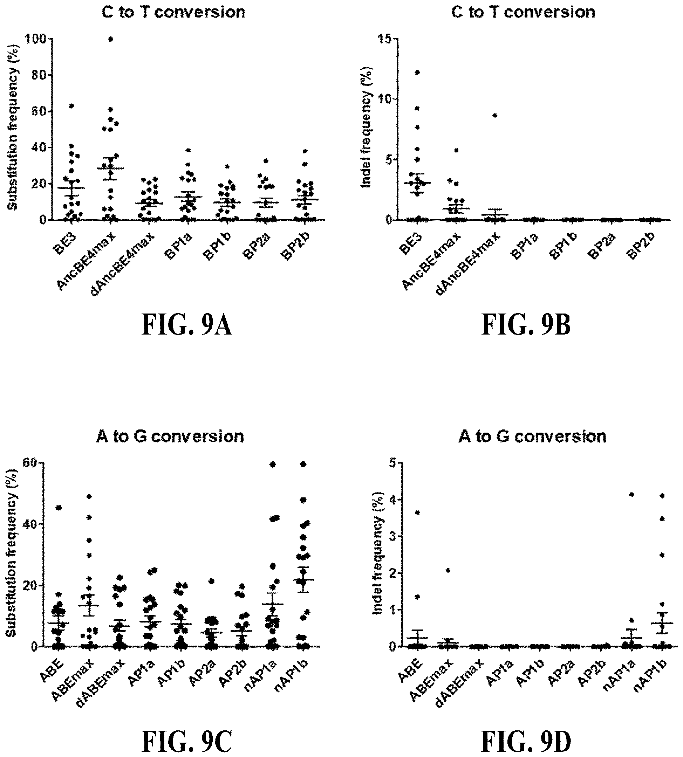

FIG. 9 illustrates confirming that undesired random indel is eliminated from a base editor including a CMP, in which FIG. 9A illustrates base substitution efficiency of CBE variants and FIG. 9B illustrates the occurrence frequency of random indel. FIG. 9C illustrates the base substitution efficiency of ABE variants, and FIG. 9D illustrates the occurrence frequency of random indel.

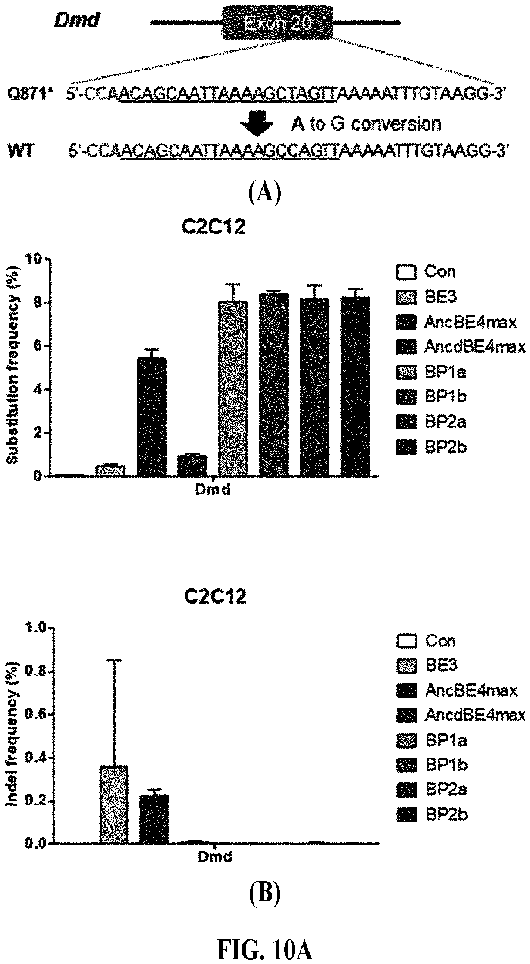

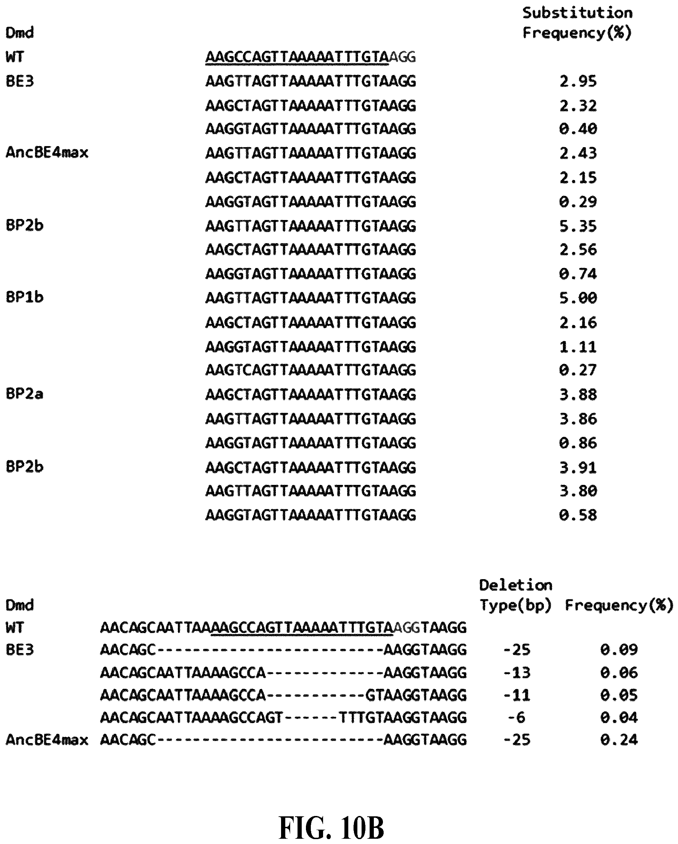

FIG. 10 illustrates results of Dmd knock-out induction experiments in mouse muscle cells and *in vivo,* in which (A) of FIG. 10A illustrates an exon20 sequence and a mutant sequence in a Dmd gene, (B) of FIG. 10A illustrates base substitution efficiency and a frequency of random indel of CBE variants including CMP, and FIG. 10B illustrates conversion patterns from C to T by the CBE variants, BE3, and AncBE4max.

### Best Mode for Carrying Out the Invention

Base editing is a genome editing method based on a clustered regular interspaced short palindromic repeats (CRISPR) system, which is widely used in various research fields. Substitution of a single base in the genome may be induced through base editing. BE3 is one of CBEs and consists of a fusion protein including nCas9, cytidine deaminase (rAPOBEC1), and an uracil DNA-glycosylase inhibitor (UGI). In addition, ABE7.10 as ABE consists of engineered homodimeric adenine deaminase TadA and nCas9, substitutes A with G in a gRNA-dependent manner. Both base editors have an active editing window, and the editing window includes a region of 13 to 17 nt upstream of a protospacer adjacent motif (PAM).

Theoretically, about 14% of pathogenic single nucleotide polymorphism (SNP) may be revised through the CBE, and about 47% thereof may be revised through the ABE. However, according to studies to date, the base editors induce random indels at a rate of 29% in mammalian cells (mouse embryos). Therefore, the elimination of occurrence of random indel by the base editor at a DNA target site remains a problem to be necessarily solved in order to apply the base editor to clinical practice.

As described above, the base editors are capable of accurate and efficient single base substitutions in the genome, but undesired insertion and deletion occur at the target site, and such a random indel limits the clinical application of the base editors. The present inventors constructed various CBE and ABE variants to eliminate random indel occurring at the target site, studied configurational elements of the base editor and the arrangement thereof to eliminate the random indel and enhance the base editing efficiency step by step, and completed the present disclosure.

First, it was confirmed that nCas9 may significantly reduce the occurrence frequency of random indel as compared to Cas9, but still generate random indel, and the random indel generated by using nCas9 may be eliminated by using dCas9. However, base editors using dCas9 have a problem in that base substitution efficiency is very low. In order to solve this problem, it is tried to improve the accessibility of the base editor to genomic DNA.

In order to improve the accessibility of the base editor to genomic DNA, a base editor variant added with a chromatin-modulating peptide (CMP) domain was constructed, and as a result of measuring the base substitution efficiency and the occurrence frequency of random indel, it was confirmed that the addition of the CMP domain varies depending on the target gene, but may enhance the base substitution efficiency and significantly reduce the occurrence frequency of random indel.

On the other hand, nCas9 in the ABE hardly generated random indel compared to nCas9 in a CBE base editor, and accordingly, an ABE variant including an additional CMP in ABEmax was constructed and the base editing efficiency and the occurrence frequency of random indel were confirmed, and as a result, it was confirmed that the base editing efficiency was significantly enhanced and the random indels were completely eliminated. From the results, it may be seen that the addition of the CMP to the base editor is effective in reducing the occurrence frequency of random indel while enhancing the base editing efficiency by increasing the accessibility to target DNA.

Accordingly, the present inventors intend to provide a fusion protein including a Cas9 protein and CMP as an enhanced base editor based on a CRISPR/Cas9 system.

In addition, the present disclosure provides a gene editing composition including the CMP-containing fusion protein and a gene-specific sgRNA (or a vector expressing the sgRNA) to be edited.

The sgRNA is single guide RNA with a length of 10 to 30 nt, preferably 19 to 30 nt, that complementarily binds to an off-target DNA strand to induce the cleavage of a target DNA strand.

In the present disclosure, "gene editing" may be used as the same meaning as gene editing or genome editing. The gene editing refers to mutations (substitution, insertion or deletion) that cause mutations of one or more bases at a target site in a target gene. Preferably, the gene editing may not involve double-stranded DNA cleavage of the target gene, and specifically, may be performed via base editing.

In one embodiment of the present disclosure, the mutation or gene editing that induces mutations for the one or more bases generates a stop codon at the target site, or generates a codon encoding an amino acid different from a wild type to knock-out the target gene. Alternatively, the mutation or gene editing may be various forms, such as knocking-out a gene or revising a genetic mutation by changing an initiation codon to another amino acid; knocking-out a gene or revising a genetic mutation by frameshift due to insertion or deletion; introducing mutations into a non-coding DNA sequence that do not produce proteins; changing DNA with a sequence different from a wild type causing a disease to the same sequence as the wild type, or the like, but is not limited thereto.

In the present disclosure, the term "base sequence" refers to a nucleotide sequence including the corresponding bases, and may be used in the same meaning as a nucleotide sequence, a nucleic acid sequence, or a DNA sequence.

In the present disclosure, the 'target gene' refers to a gene targeted for gene editing, and the 'target site or target region' refers to a site where gene editing or revising occurs by target-specific nuclease within a target gene. In one example, when the target-specific nuclease includes RNA guided engineered nuclease (RGEN), the target site may be located adjacent to a 5' end and/or 3' end of a sequence (PAM sequence) recognized by the RGEN in the target gene.

In the present disclosure, the chromatin-modulating peptides (CMPs) refer to chromosomal proteins or fragments thereof that interact with nucleosomes and/or chromosomal proteins to facilitate nucleosome rearrangement and/or chromatin remodeling. More specifically, the CMP may be high-mobility group nucleosome binding domain 1 (HN1) or a fragment thereof, histone H1 central globular domain (H1G) or a fragment thereof, or a combination thereof, but is not limited thereto.

The high-mobility group nucleosome binding domain (HMGN) is a chromosomal protein that modulates the structure and function of chromatin, and the histone H1 central globular domain (H1G) is a domain that constitutes histone H1, also referred to as 'linker histone.'. It is known that the histone H1 modulates a compaction state of a nucleosome array and affects its shape, and the central globular domain binds near the entry/exit sites of the linker DNA on the nucleosome.

The chromatin-modulating peptides may be linked to a CRISPR/Cas9 protein or reverse transcriptase directly by a chemical bond, indirectly by a linker, or in combination thereof. Specifically, at least one chromatin-modulating peptide may be linked to an N-terminus, a C-terminus, and/or an internal location of the fusion protein.

In addition, the fusion protein of the present disclosure may further include at least one nuclear localization signal, at least one cell-penetration domain, at least one marker domain, or a combination thereof, preferably further include a nuclear localization signal (NLS) sequence to an N-terminus and a C-terminus, respectively, but is not limited thereto.

In the present disclosure, the `CRISPR associated protein 9 (Cas9)' is a protein that plays an important role in the immunological defense of specific bacteria against DNA viruses, and widely used in genetic engineering applications, but may be applied to modify the genome of a cell because the main function of the protein is to cleave DNA. Specifically, CRISPR/Cas9 recognizes, cleaves, and edits a specific base sequence to be used as 3G gene scissors, and is useful for simply, quickly, and efficiently performing an operation of inserting a specific gene into a target site in the genome or stopping the activity of the specific gene. The Cas9 protein or gene information may be obtained from a known database such as GenBank of the National Center for Biotechnology Information (NCBI), but is not limited thereto. In addition, the Cas9 protein may appropriately link an additional domain by those skilled in the art depending on its purpose. In the present disclosure, the Cas9 protein may include not only wild-type Cas9 but also Cas9 variants as long as the protein has a function of nuclease for gene editing.

The Cas9 variant may be mutated to lose the activity of endonuclease for cleaving DNA double strands. For example, the Cas9 variant may be at least one selected from a Cas9 protein (nCas9) mutated to lose endonuclease activity and have nickase activity and a Cas9 protein (dCas9) mutated to lose both the endonuclease activity and the nickase activity.

The nCas9 may be deactivated by a mutation in a catalytically active domain (e.g., RuvC or HNH domain of Cas9) of the nuclease. Specifically, the nCas9 may include a mutation in which one or more selected from the group consisting of aspartic acid (D10) at position 10, glutamic acid (E762) at position 762, histidine (H840) at position 840, asparagine (N854) at position 854, asparagine (N863) at position 863 and aspartic acid (D986) at position 986 are substituted with any other amino acids. Preferably, the nCas9 of the present disclosure may include a mutation in which histidine at position 840 is substituted with alanine (H840A), but is not limited thereto.

Similarly, the dCas9 may include a mutation in which one or more selected from the group consisting of aspartic acid (D10) at position 10, glutamic acid (E762) at position 762, histidine (H840) at position 840, asparagine (N854) at position 854, asparagine (N863) at position 863 and aspartic acid (D986) at position 986 are substituted with any other amino acids. Preferably, the dCas9 of the present disclosure may include a mutation in which aspartic acid at position 10 is substituted with alanine (D10A) and a mutation in which histidine at position 840 is substituted with alanine (H840A), but is not limited thereto.

The origin of the Cas9 protein or a variant thereof is not limited, and non-limiting examples may be derived from Streptococcus pyogenes, Francisella novicida, Streptococcus thermophilus, Legionella pneumophila, Listeria innocua, or Streptococcus mutans.

The Cas9 protein or the variant thereof may be isolated from microorganisms or occur artificially or non-naturally, such as by a recombinant method or a synthetic method. The Cas9 may be used in the form of pre-transcribed mRNA or pre-produced protein in vitro, or contained in a recombinant vector for expression in a target cell or *in vivo.* In one example, the Cas9 may be a recombinant protein constructed by recombinant DNA (rDNA). The recombinant DNA refers to DNA molecules artificially constructed by genetic recombination methods such as molecular cloning to contain heterologous or homologous genetic materials obtained from various organisms.

In the present disclosure, the term "guide RNA" as used herein refers to RNA including a targeting sequence hybridizable with a specific base sequence (target sequence) within a target site in a target gene, and binds to a nuclease protein such as Cas *in vitro* or *in vivo* (or cell) to be guided to a target gene (or target site).

The guide RNA may include a spacer region (also referred to as target DNA recognition sequence, base pairing region, etc.), which is a region having a complementary sequence (targeting sequence) to a target sequence in a target gene (target site), and a hairpin structure for Cas9 protein binding. More specifically, the guide RNA may include a region having a complementary sequence to a target sequence in a target gene, a hairpin structure for Cas protein binding, and a terminator sequence.

The targeting sequence of the guide RNA capable of hybridizing with the target sequence of the guide RNA refers to a nucleotide sequence having at least 50%, at least 60%, at least 70%, at least 80%, at least 90%, at least 95%, at least 99%, or 100% of sequence complementarity with a nucleotide sequence of a DNA strand (that is, a DNA strand on which a PAM sequence (5'-NGG-3' (N is A, T, G, or C)) is located) or its complementary strand at which the target sequence is located, and is capable of complementary binding with the nucleotide sequence of the complementary strand.

The guide RNA may be used in the form of RNA (or included in the composition) or used in the form of a plasmid (or included in the composition) containing DNA encoding the RNA.

The term "chromatin accessibility" as used herein refers to a physical compaction level of chromatin which is a complex mainly formed by DNA consisting of histone, a transcription factor (TF), chromatin-modifying enzymes, and chromatin-remodeling complexes and related proteins. A eukaryotic genome is generally compacted into a nucleosome containing -147 bp of DNA surrounding a histone octamer, but the occupancy of nucleosomes is not uniform in the genome and varies according to tissue and cell types. The nucleosomes are depleted at a genomic location where cis modulatory elements (enhancers and promoters) interacting with a transcriptional modulator (ex. transcription factor) are usually present to produce an accessible chromatin. In regard to the gene revising (gene editing), since the efficiency of Cas9 is affected by local chromatin accessibility due to a significant difference in the activity of gRNA targeting an open genomic region rather than a close genomic region, it is known that there is a positive correlation between the chromatin accessibility and CRISPR-Cas9 mediated gene editing efficiency.

As another aspect of the present disclosure, the present disclosure provides a method for gene editing including bringing the gene editing composition into contact with a target region including a target nucleic acid sequence *in vitro* or *ex vivo.*

The gene editing composition may be applied to preferably eukaryotic cells, and the eukaryotic cells may be derived from preferably mammals including primates such as humans and rodents such as mice, but are not limited thereto.

As yet another aspect of the present disclosure, the present disclosure provides a gene editing kit including the gene editing composition.

In the present disclosure, the kit may include both a buffer and a material (reagent) required for performing gene editing such as deoxyribonucleotide-5-triphosphate (dNTP) together with the gene editing composition. In addition, an optimal amount of the reagent used in a specific reaction of the kit may be easily determined by those skilled in the art who have learned the disclosure herein.

As still yet another aspect of the present disclosure, the present disclosure provides a method for constructing a gene-modified mammalian animal except for human, including introducing the gene editing composition into a mammalian cell except for human to obtain a gene-modified mammalian cell; and transplanting the obtained gene-modified mammalian cell into the fallopian tube of a mammalian foster mother except to human.

In the present disclosure, the introducing of the gene editing composition into the mammalian cell may be performed by i) transfecting the cell with a plasmid vector or viral vector encoding the fusion protein according to the present disclosure and sgRNA,
ii) directly injecting a mixture of mRNA encoding the fusion protein and sgRNA or each thereof into the cell, or
iii) directly injecting a mixture of the fusion protein and sgRNA, or a ribonucleic acid protein in the form of a complex into the cell.

The direct injection may mean that each mRNA and the guide RNA or the ribonucleic acid protein of ii) or iii) passes through a cell membrane and/or nuclear membrane to be transferred to the genome without using a recombinant vector, and may be performed by, for example, nanoparticles, electroporation, lipofection, microinjection, and the like.

The mammalian cells into which the gene editing composition is introduced may be embryos of mammals including primates such as humans and rodents such as mice, and preferably embryos of mammals other than humans. For example, the embryo may be a fertilized embryo obtained by mating superovulation-induced female mammal and male mammal collected from the fallopian tube of the female mammal. The embryo to which the base revising composition is applied (injected) may be a fertilized 1-cell stage embryo (zygote).

The obtained gene-modified mammalian cell may be a cell in which a base substitution, insertion or deletion mutation has occurred in a target gene by introduction of the gene editing composition.

The mammal to which the gene-modified mammalian cell, preferably the genetically modified embryonic cell is transplanted into the fallopian tube may be a mammal (foster mother) of the same species as the mammal from which the embryonic cell is derived.

In addition, the present disclosure provides a gene-modified mammal constructed by the method.

The structure of a base editor variant designed and constructed by the present inventors and confirmed the base editing efficiency and reduced occurrence of random indel was shown in FIG. 1. Each base editor variant was constructed by adding a configuration such as CMP based on AncBE4max, which is the most optimal CBE currently known, and ABEmax, which is an ABE, and varying the arrangement thereof.

The present disclosure may have various modifications and various embodiments and specific Embodiments will be illustrated in the drawings and described in detail in the detailed description. However, the present disclosure is not limited to specific embodiments, and it should be understood that the present disclosure covers all the modifications, equivalents and replacements within the idea and technical scope of the present disclosure. In the interest of clarity, not all details of the relevant art are described in detail in the present specification in so much as such details are not necessary to obtain a complete understanding of the present disclosure.

### [Experiment method]

### 1. Cloning of plasmid vector for constructing sgRNA

An oligonucleotide specific to target sgRNA was synthesized by performing a polymerase chain reaction (PCR) using Phusion polymerase (Thermo Fisher Scientific, USA). The synthesized oligonucleotide was cloned into a pRG2-GG vector (Addgene #104174) using T4 ligase (NEB, USA). Soluble DH5a cells (Invitrogen, USA) were transfected using the cloned vector, plasmids were extracted from the transfected cells using a Midi Prep kit (MACHEREY-NAGEL, UK), and base sequences were analyzed using Sanger sequencing analysis (Macrogen, Korea).

Base sequence information of the oligonucleotide specifically synthesized for the target sgRNA and primers used for PCR was shown in Tables 1 and 2 below.

**[Table 1]**

| **Oligo Name** | **Forward (5'-3')** | **Reverse (5'-3')** |
|---|---|---|
| HBB_sgRNA | caccgCTTGCCCCACAGGGCAGTAA | aaacTTACTGCCCTGTGGGGCAA Gc |
| RNF2_sgRNA | caccgGTCATCTTAGTCATTACCTG | aaacCAGGTAATGACTAAGATGA Cc |
| HEK3_sgRNA | caccgGGCCCAGACTGAGCACGTGA | aaacTCACGTGCTCAGTCTGGGC Cc |
| Site18_sgRNA | caccgACACACACACTTAGAATCTG | aaacCTGATTCTAAGTGTGTGTGT c |
| Tyr_sgRNA | | |
| RNF2_sgRNA_gX30 | caccgCCCCTTGGCAGTCATCTTAGT CATTACCTG | aaacCAGGTAATGACTAAGATGA CTGCCAAGGGc |
| RNF2_sgRNA_gX27 | caccgCTTGGCAGTCATCTTAGTCATT ACCTG | aaacCAGGTAATGACTAAGATGA CTGCCAAGc |
| RNF2_sgRNA_gX25 | caccgTGGCAGTCATCTTAGTCATTAC CTG | aaacCAGGTAATGACTAAGATGA CTGCCAc |
| RNF2_sgRNA_gX24 | caccgGGCAGTCATCTTAGTCATTACC TG | aaacCAGGTAATGACTAAGATGA CTGCCc |
| RNF2_sgRNA_GX23 | caccgGCAGTCATCTTAGTCATTACCT G | aaacCAGGTAATGACTAAGATGA CTGCc |
| RNF2_sgRNA_GX22 | caccgCAGTCATCTTAGTCATTACCTG | aaacCAGGTAATGACTAAGATGA CTGc |
| RNF2_sgRNA_gX21 | caccgAGTCATCTTAGTCATTACCTG | aaacCAGGTAATGACTAAGATGA CTc |
| RNF2_sgRNA_gX20 | caccgGTCATCTTAGTCATTACCTG | aaacCAGGTAATGACTAAGATGA Cc |
| RNF2_sgRNA_GX19 | caccgTCATCTTAGTCATTACCTG | aaacCAGGTAATGACTAAGATGAc |
| HEK3_sgRNA_gX30 | caccgCAATCCTTGGGGCCCAGACTG | aaacTCACGTGCTCAGTCTGGGC |
| | AGCACGTGA | CCCAAGGATTGc |
| HEK3_sgRNA_gX27 | caccgTCCTTGGGCCCAGACTGAGC ACGTGA | aaacTCACGTGCTCAGTCTGGGC CCCAAGGAc |
| HEK3_sgRNA_gX25 | caccgCTTGGGGCCCAGACTGAGCAC GTGA | aaacTCACGTGCTCAGTCTGGGC CCCAAGc |
| HEK3_sgRNA_gX24 | caccgTTGGGGCCCAGACTGAGCAC GTGA | aaacTCACGTGCTCAGTCTGGGC CCCAAc |
| HEK3_sgRNA_gX23 | caccgTGGGGCCCAGACTGAGCACG TGA | aaacTCACGTGCTCAGTCTGGGC CCCAc |
| HEK3_sgRNA_gX22 | caccgGGGGCCCAGACTGAGCACGT GA | aaacTCACGTGCTCAGTCTGGGC CCCc |
| HEK3_sgRNA_GX21 | caccgGGGCCCAGACTGAGCACGTG A | aaacTCACGTGCTCAGTCTGGGC CCc |
| HEK3_sgRNA_GX20 | caccgGGCCCAGACTGAGCACGTGA | aaacTCACGTGCTCAGTCTGGGC Cc |
| HEK3_sgRNA_GX19 | caccgGCCCAGACTGAGCACGTGA | aaacTCACGTGCTCAGTCTGGGCc |

| **1st PCR primer name** | **Forward (5'-3')** | **Reverse (5'-3')** |
|---|---|---|
| HBB_deep seq_1st | ACTGTGTTCACTAGCAACCTC | TGATGCAATCATTCGTCTGTTTC |
| RNF2_deep seq_1st | TGTCAGAACATGCTGGAAGG | AGGACTTGCCCAACTTTCTAC |
| HEK3_deep seq_1st | TGGGTCACAGTGGCAAAT | GGGTAATCTGGTTGATCTCTGAT |
| Site18_deep seq_1st | AGAAACACCTTGGAGGAAGTG | CCAGTTAAGGAGAGGAATGGA AA |
| Tyr_deep seq_1st | TGTATTGCCTTCTGTGGAGTT | GGTGTTGACCCATTGTTCATTT |

| **2nd PCR primer name** | **Adaptor sequence + Sequence (5'-3')** | |
|---|---|---|
| HBB_deep seq_2nd_F | ACACTCTTTCCCTACACGACGCTCTTCCGATCTCACTAGCAACCTCAA ACAGACA | |
| HBB_deep seq_2nd_R | GTGACTGGAGTTCAGACGTGTGCTCTTCCGATCTGTGCCTATCAGAA ACCCAAGAG | |
| RNF2_deep seq_2nd_F | ACACTCTTTCCCTACACGACGCTCTTCCGATCTTGCAGACAAACGGA ACTCAA | |
| RNF2_deep seq_2nd_R | GTGACTGGAGTTCAGACGTGTGCTCTTCCGATCTGCCAACATACAGA AGTCAGGAA | |
| HEK3_deep seq_2nd_F | ACACTCTTTCCCTACACGACGCTCTTCCGATCTCCAAACTTGTCAACC AGTATCC | |
| HEK3_deep seq_2nd R | GTGACTGGAGTTCAGACGTGTGCTCTTCCGATCTGTCTATTTCTGCTG CAAGTAAGC | |
| Site18_deep seq_2nd F | ACACTCTTTCCCTACACGACGCTCTTCCGATCTTGCTGTCCAAGAAGC AACA | |
| Site18_deep seq_2nd R | GTGACTGGAGTTCAGACGTGTGCTCTTCCGATCTACCTCTAGTTCTCA AACTTCAGC | |
| Tyr_deep seq_2nd F | ACACTCTTTCCCTACACGACGCTCTTCCGATCTGGAGTTTCCAGATCT CTGATGG | |
| Tyr_deep seq_2nd_R | GTGACTGGAGTTCAGACGTGTGCTCTTCCGATCTGCACTGGCAGGTC CTATTAT | |

**[Table 2]**

| **Oligo Name** | **Forward (5'-3')** | **Reverse (5'-3')** |
|---|---|---|
| Site18_sgRNA | caccgACACACACACTTAGAATCTG | aaacCTGATTCTAAGTGTGTGTGTc |
| Site19_sgRNA | caccgCACACACACTTAGAATCTGT | aaacACAGATTCTAAGTGTGTGTGc |
| HBB-E2_sgRNA | caccgTCAGAAAGTGGTGGCTGGT G | aaacCACCAGCCACCACTTTCTGAc |
| HEK2_sgRNA | caccgGAACACAAAGCATAGACTG C | aaacGCAGTCTATGCTTTGTGTTCc |
| Site 18_sgRNA_gX30 | caccgACACACAGACACACACACA CTTAGAATCTG | aaacCAGATTCTAAGTGTGTGTGTG TCTGTGTGTc |
| Site 18_sgRNA_gX27 | caccgCACAGACACACACACACTT AGAATCTG | aaacCAGATTCTAAGTGTGTGTGTG TCTGTGc |
| Site 18_sgRNA_gX25 | caccgCAGACACACACACACTTAG AATCTG | aaacCAGATTCTAAGTGTGTGTGTG TCTGc |
| Site 18_sgRNA_gX24 | caccgAGACACACACACACTTAGA ATCTG | aaacCAGATTCTAAGTGTGTGTGTG TCTc |
| Site 18_sgRNA_gX23 | caccgGACACACACACACTTAGAAT CTG | aaacCAGATTCTAAGTGTGTGTGTG TCc |
| Site 18_sgRNA_GX22 | caccgACACACACACACTTAGAATC TG | aaacCAGATTCTAAGTGTGTGTGTG Tc |
| Site 18_sgRNA_gX21 | caccgCACACACACACTTAGAATCT G | aaacCAGATTCTAAGTGTGTGTGTG c |
| Site 18_sgRNA_gX20 | caccgACACACACACTTAGAATCTG | aaacCAGATTCTAAGTGTGTGTGTc |
| Site 18_sgRNA_gX19 | caccgCACACACACTTAGAATCTG | aaacCAGATTCTAAGTGTGTGTGc |
| HEK2_sgRNA_gX30 | caccgAAGGAAACTGGAACACAAA GCATAGACTGC | aaacGCAGTCTATGCTTTGTGTTCC AGTTTCCTTc |
| HEK2_sgRNA_GX27 | caccgGAAACTGGAACACAAAGCA TAGACTGC | aaacGCAGTCTATGCTTTGTGTTCC AGTTTCc |
| HEK2_sgRNA_gX25 | caccgAACTGGAACACAAAGCATA GACTGC | aaacGCAGTCTATGCTTTGTGTTCC AGTTc |
| HEK2_sgRNA_gX24 | caccgACTGGAACACAAAGCATAG ACTGC | aaacGCAGTCTATGCTTTGTGTTCC AGTc |
| HEK2_sgRNA_gX23 | caccgCTGGAACACAAAGCATAGA CTGC | aaacGCAGTCTATGCTTTGTGTTCC AGc |
| HEK2_sgRNA_gX22 | caccgTGGAACACAAAGCATAGAC TGC | aaacGCAGTCTATGCTTTGTGTTCC Ac |
| HEK2_sgRNA_gX21 | caccgGGAACACAAAGCATAGACT GC | aaacGCAGTCTATGCTTTGTGTTCCc |
| HEK2_sgRNA_GX20 | caccgGAACACAAAGCATAGACTG C | aaacGCAGTCTATGCTTTGTGTTCc |
| HEK2_sgRNA_gX19 | caccgAACACAAAGCATAGACTGC | aaacGCAGTCTATGCTTTGTGTTc |

| 1st **PCR primer name** | **Forward (5'-3')** | Reverse (5'-3') |
|---|---|---|
| Site18_deep seq_1st | AGAAACACCTTGGAGGAAGTG | CCAGTTAAGGAGAGGAATGGAA A |
| Site19_deep seq_1st | AGAAACACCTTGGAGGAAGTG | CCAGTTAAGGAGAGGAATGGAA A |
| HBB-E2_deep seq_1st | CTCTTTCTTTCAGGGCAATAATGA TAC | GGCAGAATCCAGATGCTCAA |
| HEK2_deep seq_1st | TCTAGAGGTCCTAAACCAGTGT | CCTCAGCATTCAGCCACTAATA |

| **2nd PCR primer name** | **Adaptor sequence** + **Sequence** (5'-3') | |
|---|---|---|
| Site18_deep seq_2nd_F | ACACTCTTTCCCTACACGACGCTCTTCCGATCTTGCTGTCCAAGAAGC AACA | |
| Site18_deep seq_2nd_R | GTGACTGGAGTTCAGACGTGTGCTCTTCCGATCTACCTCTAGTTCTCA AACTTCAGC | |
| Site19_deep seq_2nd_F | ACACTCTTTCCCTACACGACGCTCTTCCGATCTTGCTGTCCAAGAAGC AACA | |
| Site19_deep seq_2nd_R | GTGACTGGAGTTCAGACGTGTGCTCTTCCGATCTACCTCTAGTTCTCA AACTTCAGC | |
| HBB-E2_deep seq_2nd_F | ACACTCTTTCCCTACACGACGCTCTTCCGATCTACCTCTTATCTTCCTC CCACA | |
| HBB-E2_deep seq_2nd_R | GTGACTGGAGTTCAGACGTGTGCTCTTCCGATCTAGTTGGACTTAGG GAACAAAGG | |
| HEK2_deep seq_2nd_F | ACACTCTTTCCCTACACGACGCTCTTCCGATCTGGACGTCTGCCCAAT ATGTAA | |
| HEK2_deep seq_2nd_R | GTGACTGGAGTTCAGACGTGTGCTCTTCCGATCTCATCTGTCAAACTG TGCGTATG | |

### 2. Preparation of vector for base editing

xCas9 (3.7)-BE3 (Addgene #108380), pCMV-BE3 (Addgene #73021), pCMV-AncBE4max (Addgene #112094), xCas9(3.7)-ABE7.10 (Addgene #108382), pCMV-ABE7.10 (Addgene #102919), and pCMV-ABEmax (Addgene #112095) were constructed from Addgene and a pCMV-NLS-UGI vector was constructed from GeneCker, Inc. (Korea).

### 3. Cell culture and transfection

HEK293T cells (ATCC CRL-3216) were incubated in a Dulbecco modified Eagle's medium (DMEM; Welgene, Korea) supplemented with 10% fetal bovine serum (FBS; Gibco, USA) at 37°C and 5% CO₂. The incubated cells were inoculated in a 24-well plate (SPL, Korea) at a concentration of 2 × 10⁴ per well, and after 17 hours, transfected with a base editor plasmid (750 ng), a sgRNA plasmid (250 ng), or a UGI plasmid (250 ng or 500 ng) according to a manufacturer's protocol using 1 µl of Lipofectamine 2000 (Thermo Fisher Scientific, USA). After 72 hours of transfection, the cells were harvested and lysed to be used as PCR templates.

### 4. Preparation of mRNA

pET-AncBE4max and pET-UGI were constructed by GeneCker, Inc (Korea).

Each mRNA template was constructed through PCR using Phusion polymerase (Thermo Fisher Scientific, USA). Primer sequences performed for PCR were shown in Table 3 below.

**[Table 3]**

| | |
|---|---|
| Primer_F | 5'-GGT GAT GTC GGC GAT ATA GG-3' |
| Primer_R | 5'-CCC CAA GGG GTT ATG CTA GT-3' |

The mRNA was prepared using an RNA transcription kit (mMESSAGE mMACHINE T7 Ultra kit, Ambion) and purified using a MEGAclear kit (Ambion).

### 7. Targeted deep sequencing

A target region was amplified from genomic DNA using Phusion polymerase (Thermo Fisher Scientific, USA) and a PCR thermal cycler. PCR amplicons were subjected to paired-end sequencing using an Illumina MiSeq system (Illumina, Inc., USA). The primers used were shown in Table 1 above. Targeted deep sequencing data were analyzed using CRISPR RGEN Tools (www.rgenome.net) and EUN program (daeunyoon.com).

### 8. Statistical analysis

Data were analyzed using SPSS software, version 18.0 (SPSS Inc., Chicago, IL, USA). P values were determined by performing unpaired and two-sided Student's t-tests or One-way ANOVA, and performing Tukey by (multiple comparisons) post-hoc. All data were expressed as mean and standard deviation (S.D.).

### [Experimental Results]

### Embodiment 1. Confirmation of induction of undesired indel at DNA target site by base editor variant

Several types of base editor variants based on a CRISPR system have been developed for more precise and efficient genetic manipulation. Among the developed base editor variants, AncBE4max and ABEmax were optimized by modulating configurational elements of nuclear localization signals (NLS), codons, and deaminase, and the use thereof. Such modulation significantly improved the efficiency and accuracy of the base editors in cells to enable precise SNP modifications. In addition, xCas9-BE3 (xBE3) and xCas9-ABE (xABE) were advanced base editors fused with xCas9 (3.7), and base editors with improved compatibility with a wide range of PAMs by recognizing NG or NGT PAM sequences.

The present inventors constructed various variants of the base editors and tried to confirm the efficiency of base substitution and insertion/deletion of the variants in human HEK293T cells. xBE3, BE3, and AncBE4max were constructed as CBE variants, and AncBE4max, xABE, ABE, and ABEmax were constructed as ABE variants (FIG. 1), CBE target loci were human *HBB, RNF2, HEK3,* and *Site18,* and ABE target loci were human *Site18, Site19, HBB-E2* and *HEK22.*

As a result, it was confirmed that the base substitution efficiency of AncBE4max and ABEmax was higher than that of other base editors (FIG. 2). All of the ABE and CBE variants induced A to G or C to T base substitution at all target loci (FIG. 3). Among the CBE variants, AncBE4max showed substitution efficiency of up to 89% at the target site, but showed lower insertion/deletion efficiency than BE3 (FIG. 3A). Insertion and deletion at the *HEK3* target site for BE3 were confirmed up to 6.5% (FIG. 3B). Unlike AncBE4max, ABEmax showed higher levels of substitution and insertion/deletion than other base editors (FIGS. 3C and 3D). Base editors xBE3 and xABE showed the lowest insertion/deletion and substitution efficiency at all target sites, which was considered to be caused by the low activity of xCas9 (3.7). The ABE variant had lower relative indel efficiency than the CBE variant, and indels rarely occurred depending on the target sequence. From the results, it may be seen that the base editor variants may induce undesired insertion and/or deletion of bases at a DNA target site with high efficiency.

### Embodiment 2. Confirmation of effect of sgRNA length on base substitution efficiency and occurrence of random indel

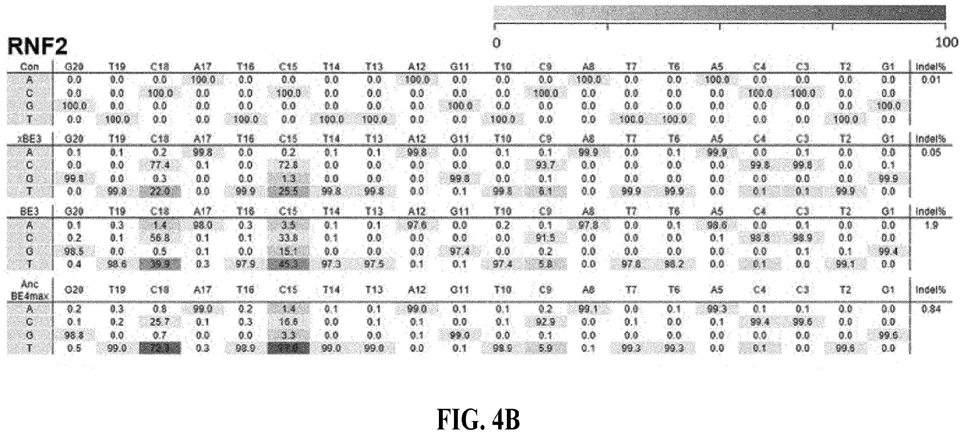

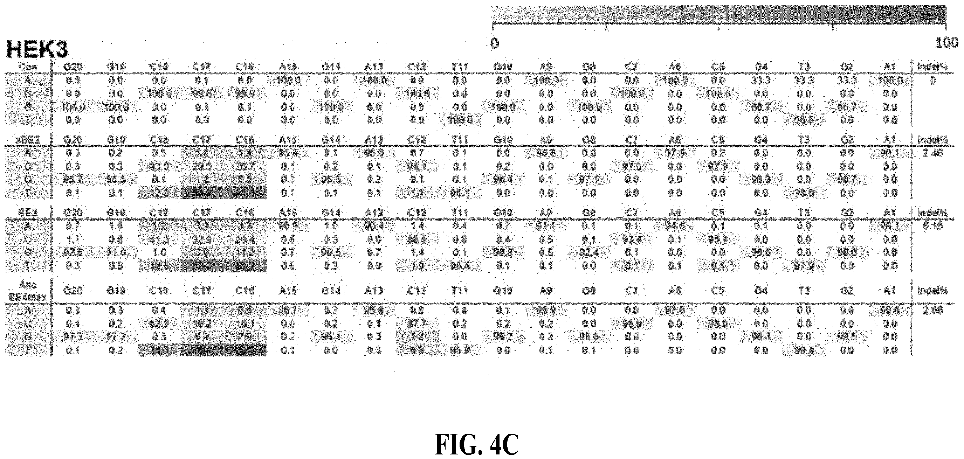

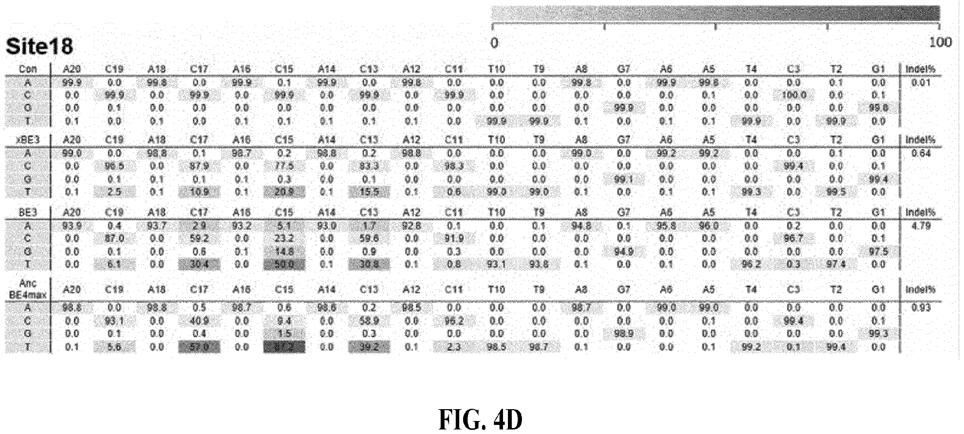

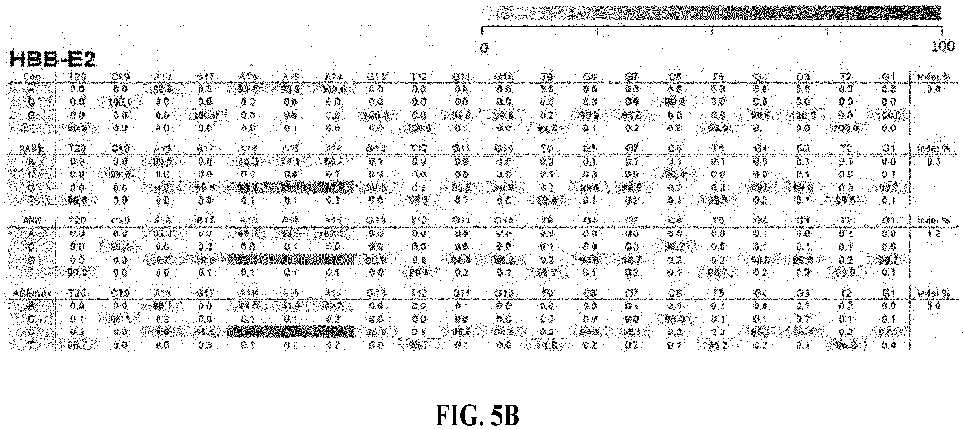

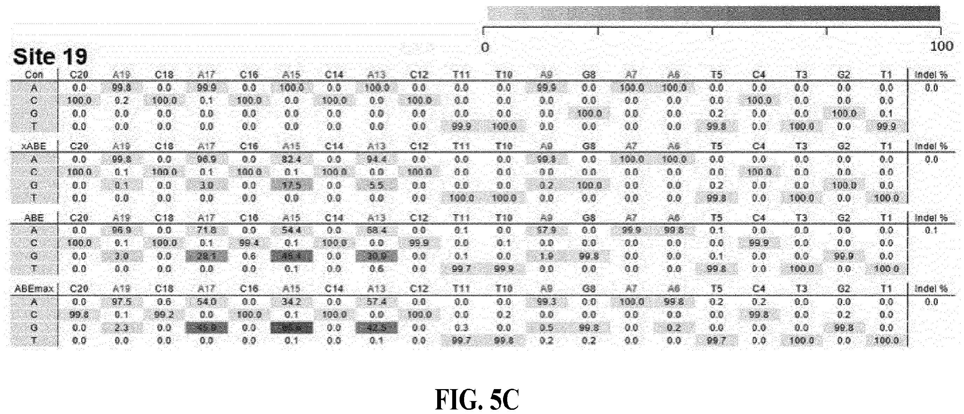

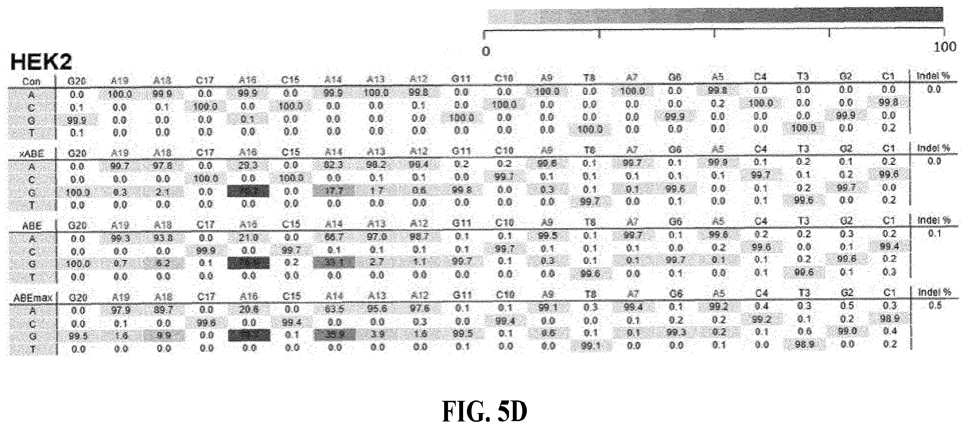

Subsequently, the present inventors attempted to confirm the effect of the length of single guide RNA (sgRNA) on the indel efficiency of a base editor. Specifically, by extending 19 to 30 bases to each target site, sgRNAs with different lengths were constructed, indel efficiency was confirmed, and simultaneously, base editing efficiency, specificity, and editing windows of CBE and ABE variants at the target site were compared with each other (FIGS. 4 and 5).

As a result, the substitution frequency in *HEK3* and *RNF2* was highest in Gx19 sgRNA, and the occurrence frequency of undesired insertion and deletion was lowest in gx30 sgRNA (FIG. 6). In addition, the sgRNA extended from the CBE variant extended the active editing window of the target sequence compared to the ABE variant, but it was confirmed that there was no effect on the location of the cleavage site.

In addition, while ABE and ABEmax showed similar substitution efficiencies in almost all sgRNA lengths, it was confirmed that the substitution efficiency of xABE increased as the sgRNA length became shorter (FIG. 6).

The indel efficiency of target-specific ABE variants differed slightly depending on the sgRNA length, but its difference was less than 1%. Also, unlike the CBE variant, the ABE variant consistently showed a stable and narrow editing window regardless of the length of the sgRNA. In addition, the sgRNA of the ABE variant was confirmed to have increased target specificity in all sgRNA lengths except gx21 (FIG. 6).

From the results, it may be seen that higher base substitution efficiency and undesired base insertion and/or deletion may be excluded through the modulation of the sgRNA length of the ABE and CBE variants.

### Embodiment 3. Confirmation of reduction in occurrence frequency of random indel by CBE according to UGI treatment

The occurrence frequency of insertion/deletion induced by nCas9 was up to 6.5% depending on the target, and the insertion/deletion was mainly induced around upstream 3 nucleotides of the PAM sequence, which was a Cas9-dependent target cleavage site (FIG. 7).

The present inventors have confirmed that undesired base insertion and deletion caused by base editor variants was sufficiently eliminated by using dCas9 linked with deaminase. However, when dCas9 was used instead of nCas9 in the base editor, the occurrence of undesired indel may be completely eliminated in both CBE and ABE variants, but there was a problem in that the efficiency of base editing was remarkably low (FIGS. 7C to 7F). In particular, when targeting RNF2 depending on the target, the editing efficiency of AncdBE4max among CBE variants was reduced by about 9.5 times compared to AncBE4max.

### Embodiment 4. Confirmation of reduction in occurrence frequency of random indel in CBE using dCas9

As confirmed in Embodiment 3 above, indel was reduced due to CBE and ABE variants by using dCas9 instead of nCas9 in the CBE base editor using dCas9, but dCas9 showed very low base substitution efficiency in both variants (FIG. 1). In order to overcome low editing efficiency, the present inventors tried to increase the accessibility of Cas9 to the target sequence by adding a chromatin modulating peptide (CMP) domain to the base editor. Base editor variants were constructed by disposing a high-mobility group nucleosome binding domain 1 (HN1) and a histone H1 central globular domain (H1G) in various regions (FIG. 8). For human targets, the base editing efficiency and the occurrence frequency of indel according to the order of CMP configurational elements were confirmed between each CBE and ABE (FIG. 9).

All the BP and AP variants containing dCas9 eliminated the occurrence of indel, and as a result, it was confirmed that more accurate base editing was enabled without undesired base insertion and deletion. Specifically, BPla and BP2b exhibited lower base substitution efficiency than AncBE4max, but had higher substitution efficiency than dAncBE4max and did not generate indels. In addition, APla and APlb showed slightly higher editing efficiency than dABEmax, but ABEmax had a low occurrence frequency of indel, so that nCas9 was used for APla and APlb (nAP1a and nAP1b). As a result, nAPlb showed significantly enhanced base substitution efficiency at the target site.

BE3 variants targeting *HEK3* and *Site18* did not have a significant reduction in occurrence frequency of indel due to increased chromatin accessibility by targeting an open chromatin structure.

### Embodiment 5. Construction of Dmd KO animal model using base editor variant and confirmation of base editing efficiency

Duchenne muscular dystrophy (DMD) was a genetic disorder found in one of 3500 to 5000 men, and caused muscle weakness and degeneration as genetic disease and caused by the breakdown of dystrophin (Dmd).

The present inventors designed an sgRNA specific to exon 20 of Dmd to make a pre-stop codon (CAG > TAG) and compared C to T substitution ability of CBE and BP variants in mouse myoblasts (C2C12), and all BP variants confirmed high editing ability and elimination efficacy of undesired occurrence of indel (FIGS. 10 and 11).

Specifically, AncBE4max and BP2b were packaged into lentiviruses together with sgRNAs with a mouse nontarget (MNT) sequence or Dmd (plenti-MNT-AncBE4max, plenti-Dmd-AncBE4max, plenti-Dmd-BP2b) as controls. Gene editing was confirmed by injecting base editor-packed lentivirus into P1 to P3 of C57BL/6N mice (5 × 10⁵ TU/TA muscle) and performing NGS and histological analysis after 1, 3, and 6 months.

As a result, it was confirmed that when a stop codon was generated in a Duchenne muscular dystrophy (Dmd) gene of the muscle cell line C2C12 using the CBE variant, both BPla and AP2b induced C to T conversion with higher efficiency than BE3 and AncBE4max, and undesired indel did not occur (FIG. 10).

In addition, the Dmd mutant (Q863*) C2C12 was also transfected with the ABE and AP variants. AP2b or nAPlb showed the highest efficiency in A to G substitution capable of restoring previously stopped translation to a normal. plenti-MNT-ABEmax, plenti-Dmd rescue-ABEmax, plenti-Dmd rescue-AP2b or nAPlb was packed in lentivirus and injected at birth in Dmd mutant (Q863*) mice at the same titer as CBE (P1 to P3). NGS and histological analyzes were performed after 1, 3, and 6 months of injection to confirm gene editing.

As described above, specific parts of the present disclosure have been described in detail, and it will be apparent to those skilled in the art that these specific techniques are merely preferred example embodiments, and the scope of the present disclosure is not limited thereto. Therefore, the substantial scope of the present disclosure will be defined by the appended claims and their equivalents.

## Claims

1. A gene editing fusion protein comprising:
a Cas9 protein; and
one or more chromatin-modulating peptides (CMPs).

2. The gene editing fusion protein of claim 1, wherein the CMP is at least one selected from the group consisting of a high-mobility group nucleosome binding domain 1 (HN1), a histone H1 central globular domain (H1G), and a combination thereof.

3. The gene editing fusion protein of claim 1, wherein the fusion protein further comprises cytosine deaminase, and
the Cas9 protein is dead Cas9 (dCas9).

4. The gene editing fusion protein of claim 1, wherein the fusion protein further comprises tRNA adenosine deaminase (TadA), and
the Cas9 protein is nickase Cas9 (nCas9) or dead Cas9 (dCas9).

5. The gene editing fusion protein of claim 3 or 4, wherein the fusion protein further comprises a nuclear localization signals (NLS) peptide.

6. The gene editing fusion protein of claim 5, wherein [NLS peptide]-[Cas9 protein]-[NLS peptide] are located in the order from an N-terminus to a C-terminus of the fusion protein, and
the CMP is located between the C-terminus of the fusion protein and/or the NLS peptide and the Cas9 protein.

7. The gene editing fusion protein of claim 6, wherein [NLS peptide]-[HN1]-[Cas9 protein]-[H1G]-[NLS peptide] are located in the order from the N-terminus to the C-terminus of the fusion protein, and
[NLS peptide]-[HN1]-[Cas9 protein]-[NLS peptide]-[H1G] are located in the order from the N-terminus to the C-terminus.

8. The gene editing fusion protein of claim 3, wherein the fusion protein further comprises one or more uracil DNA-glycosylase inhibitor (UGI) peptides.

9. The gene editing fusion protein of claim 8, wherein the UGI peptide is directly linked to the C-terminus of the dCas9 protein.

10. The gene editing fusion protein of claim 9, wherein [NLS peptide]-[HN1]-[dCas9 protein]-[UGI peptide]-[UGI peptide]-[NLS peptide]-[H1G] are located in the order from the N-terminus to the C-terminus of the fusion protein, or
[NLS peptide]-[HN1]-[dCas9 protein]-[UGI peptide]-[UGI peptide]-[NLS peptide]-[H1G] are located in the order from the N-terminus to the C-terminus.

11. A gene editing composition comprising:
the fusion protein of claim 1, plasmid DNA or mRNA encoding the fusion protein, or a vector including the plasmid DNA or mRNA; and
single guide RNA (sgRNA) hybridizing with an off-target DNA strand to induce cleavage of the target DNA strand, a plasmid capable of expressing the sgRNA, or a vector including the plasmid.

12. The gene editing composition of claim 11, wherein the composition further comprises UGI.

13. A method for gene editing comprising bringing the gene editing composition of claim 11 into contact with a target region including a target nucleic acid sequence in vitro or ex vivo.

14. A lentiviral vector comprising mRNA encoding the fusion protein of claim 1 and single guide RNA (sgRNA).

15. The lentiviral vector of claim 14, wherein the sgRNA has a length of 10 to 30 nucleotides (nt).

16. A method for constructing a transfected cell line comprising bringing the lentiviral vector of claim 14 into contact with a mammalian cell.

17. A transfected cell line infected with the lentiviral vector of claim 14.
